(19) Europäisches Patentamt
European Patent Office
Office européen des brevets

(11) Veröffentlichungsnummer : **0 492 105 B1**

(12) **EUROPÄISCHE PATENTSCHRIFT**

(45) Veröffentlichungstag der Patentschrift :
**20.07.94 Patentblatt 94/29**

(51) Int. Cl.[5] : **A61K 31/415, A61K 31/41**

(21) Anmeldenummer : **91119183.1**

(22) Anmeldetag : **11.11.91**

(54) Angiotensin-II-Receptoren-Blocker zur Behandlung der cardialen Hypertrophie.

(30) Priorität : **17.11.90 DE 4036706**

(43) Veröffentlichungstag der Anmeldung :
**01.07.92 Patentblatt 92/27**

(45) Bekanntmachung des Hinweises auf die
Patenterteilung :
**20.07.94 Patentblatt 94/29**

(84) Benannte Vertragsstaaten :
**AT BE CH DE DK ES FR GB GR IT LI LU NL SE**

(56) Entgegenhaltungen :
EP-A- 0 028 833
EP-A- 0 450 566
EP-A- 0 459 136
EP-A- 0 468 372
EP-A- 0 485 929
US-A- 4 880 804
HYPERTENSION, Band 18, Nr. 4, Oktober 1991,
Suppl., Seiten II-100 - II-105, & THE RENIN-
ANGIOTENSIN SYSTEM AND THE VASCULAR
WALL: FROM EXPERIMENTAL MODELS TO
MAN, Monta Carlo, 12. - 13. Oktober 1990; V.J.
DZAU et al.: "Molecular mechanisms of vascular renin-angiotensin system in myointimal
hyperplasia"
JOURNAL OF HYPERTENSION, Band 9, Suppl.
6, 1991, Seiten 5226-5227, Current Science Ltd;
A. SACHINIDIS et al.: "Effect of the angiotensin II receptor antagonist MK 954 on the angiotensin II-induced increase in free cytosolic
Ca2+ and growth in vascular smooth muscle
cells"
JOURNAL OF HYPERTENSION, Band 9, Suppl.
6, 1991, Seiten 5400-5401, Current Science Ltd;
W. LINZ et al.: "Role of angiotensin II receptor
antagonism and converting enzyme inhibition
in the progression and regression of cardiac
hypertrophy in rats"

(56) Entgegenhaltungen :
JOURNAL OF CLINICAL HYPERTENSION,
Band 3, 1987, Seiten 87-103, Elsevier Science
Publishing Co., Inc., New York, US; R.B. DEVE-
REUX et al.: "Relation of renin-angiotensin
system activity to left ventricular hypertrophy
and function in experimental and human hypertension"

(73) Patentinhaber : **HOECHST
AKTIENGESELLSCHAFT
D-65926 Frankfurt (DE)**

(72) Erfinder : **Heitsch, Holger, Dr.
Martin-Wohmann-Strasse 27
W-6238 Hofheim am Taunus (DE)**
Erfinder : **Henning, Rainer, Dr.
Im Höhlchen 16
W-6234 Hattersheim am Main (DE)**
Erfinder : **Linz, Wolfgang, Dr.
Huxelrebenweg 54
W-6500 Mainz (DE)**
Erfinder : **Schölkens, Bernward, Prof.Dr.
Hölderlinstrasse 62
W-6233 Kelkheim/Taunus (DE)**
Erfinder : **Urbach, Hansjörg, Dr.
Le Lavandoustrasse 41
W-6242 Kronberg/Taunus (DE)**

**Beschreibung**

Die Erfindung betrifft die Verwendung von Angiotensin-II-Rezeptoren-Blockern zur Herstellung von Arzneimitteln zur Behandlung der cardialen Hypertrophie.

Aus den EP-A 028 833, EP-A 028 834, EP-A 323 841, EP-A 324 377, EP-A 291 969, US-A 4 355 040, US-A 4 880 804 und US-A 4 916 129 sowie aus J. Med. Chem., Bd. 33, 1312 (1990); Hypertension, Bd. 13, 489 (1989); Hypertension, Bd. 14, 348 (1989); Hypertension, Bd. 15, 459 (1990); Cardiov. Drug Rev., Bd. 7, 285 (1989); Mol. Pharmacol., Bd. 37, 347 (1990); J. Pharmacol. Exp. Ther., Bd. 250,515 (1989); J. Pharmacol. Exp. Ther., Bd. 250, 867 (1989); J. Pharmacol. Exp. Ther., Bd. 252, 711 (1990); J. Pharmacol. Exp. Ther., Bd. 252, 719 (1990) und J. Pharmacol. Exp. Ther., Bd. 252, 726 (1990) sind substituierte Imidazole, Pyrrole, Pyrazole und Triazole als Antihypertensiva und Mittel zur Behandlung der Herzinsuffizienz bekannt.

In J. Clin. Hypertension, 3: 1987, 87-103 wird der Einfluß des Renin-Angiotensin Systems auf Blutdruck und kardiale Hypertrophie untersucht. Die nicht vorveröffentlichten älteren EP-A 485929, EP-A 468372, EP-A 459136 und EP-A 450566 beschreiben substituierte Azolderivate und Benzimidazolderivate als Antihypertensiva und Angiotensin II-Antagonisten und deren Verwendung als Mittel zur Behandlung von Hypertensie und Herzerkrankungen.

Die nachveröffentlichte Schrift Hypertension, 18 (4), Suppl., Oktober 1991, S. II-100 bis 11-105 beschreibt Imidazolverbindungen, die zur Behandlung von vaskulären Erkrankungen verwendet werden können.

Es wurde nun überraschend gefunden, daß Verbindungen dieser Strukturtypen darüber hinaus hochwirksame und hochspezifische Cardioprotektiva sind, die die Hypertrophie des Herzmuskels aufzuheben vermögen.

Bevorzugt für ihre cardioprotektive Wirkung sind Verbindungen vom Imidazoltyp sowie deren physiologisch verträglichen Salze.

Unter physiologisch verträglichen Salzen von genannten Verbindungen versteht man sowohl deren organische als auch anorganische Salze, wie sie in Remington's Pharmaceutical Sciences, 17. Auflage, Seite 1418 (1985) beschrieben sind. Aufgrund von physikalischer und chemischer Stabilität und Löslichkeit sind für saure Gruppen unter anderem Natrium-, Kalium-, Calcium- und Ammoniumsalze bevorzugt; für basische Gruppen unter anderem Salze mit Salzsäure, Schwefelsäure, Phosphorsäure, Kohlensäure oder Sulfonsäuren, sowie Essigsäure, Citronensäure, Benzoesäure, Maleinsäure, Fumarsäure, Weinsäure, p-Toluolsulfonsäure.

Besonders bevorzugt für ihre cardioprotektive Wirkung sind die folgenden 4 Verbindungen:

1. 2-n-Butyl-4-chloro-5-hydroxymethyl-1-[(2'-(1H-tetrazol-5-yl)-biphenyl-4-yl)-methyl]-imidazol,
2. 2-n-Butyl-5-carboxy-4-chloro-1-[(2'-(1H-tetrazol-5-yl)-biphenyl-4-yl)-methyl]-imidazol,
3. 2-n-Butyl-1-[(2'-carboxy-biphenyl-4-yl)-methyl]-4-chloro-5-hydroxymethyl-imidazol,
4. 2-n-Butyl-5-carboxy-1-[(2'-carboxy-biphenyl-4-yl)-methyl]-4-chloro-imidazol-.

sowie deren physiologisch verträglichen Salze.

In Ausübung der erfindungsgemäßen Methode können die oben beschriebenen Antagonisten des Angiotensin-II-Rezeptors an Säugern wie Affen, Hunden, Katzen, Ratten, Menschen etc. angewendet werden. Die für die erfindungsgemäße Verwendung geeigneten Verbindungen werden zweckmäßig in üblicher Weise in pharmazeutische Präparate eingearbeitet. Sie können in die üblichen Verabreichungsformen wie Kapseln, Tabletten, Dragees, Lösungen, Salben, Emulsionen und auch in Depot-Form gebracht werden. Der Wirkstoff kann gegebenenfalls auch in mikroverkapselter Form vorliegen. Die Präparate können physiologisch verträgliche organische bzw. anorganische Hilfs- bzw. Zusatzstoffe, beispielsweise Granulierstoffe, Kleb- und Bindemittel, Gleitmittel, Suspendiermittel, Lösungsmittel, antibakterielle Mittel, Netzmittel und Konservierungsmittel enthalten.

Die erfindungsgemäße Behandlung kann sowohl über die Schleimhäute als auch parenteral erfolgen. Orale und parenterale (wie i.v. oder i.m.) Anwendungsformen werden bevorzugt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträglichen Salze gewünschtenfalls mit den dafür üblichen Substanzen wie Lösungsvermittler, Emulgatoren oder weitere Hilfsstoffe in Lösung, Suspension oder Emulsion gebracht. Als Lösungsmittel für die aktiven Kombinationen und die entsprechenden physiologisch verträglichen Salze kommen z. B. in Frage: Wasser, physiologische Kochsalzlösungen oder Alkohole, z. B. Ethanol. Propandiol oder Glycerin, daneben auch Zuckerlösungen wie Glucose- oder Mannitlösungen oder auch eine Mischung aus den verschiedenen genannten Lösungsmitteln.

Die Verbindungen werden bevorzugt in Dosen von 0,1 bis 100 mg/kg verabreicht, wobei im einzelnen vorzugsweise 0,1 bis 50 mg, insbesondere 1 bis 30 mg ein- bis dreimal täglich gegeben werden.

Versuch

Wirkung des Angiotensin-II-Rezeptor-Antagonisten 2-n-Butyl-4-chloro-5-hydroxymethyl-1-[(2'-(1H-tetrazol-5-yl)-biphenyl-4-yl)methyl]-imidazol-Kaliumsalz (Verbindung III) auf Entwicklung und Regression der cardialen Hypertrophie bei Ratten

Methode

Bei wachen Ratten wurde durch Einengung der abdominalen Aorta eine Herzhypertrophie erzeugt. Nachdem diese voll etabliert war, erhielten Gruppen der Tiere für 6 Wochen 3 mg/kg der Verbindung III durch Gabe über ihr Trinkwasser. Im Preventationsexperiment begann die Gabe des Antagonisten sofort, im Regressionsexperiment hingegen erst 6 Wochen nach dem Setzen der Aortenstennose. Kontrollgruppen ohne Substanzgabe (CON) und scheinoperierte Tiere (SHAM) wurden mitgeführt. Nach Ende des Beobachtungszeitraums wurden die Tiere getötet und das Herzgewicht, die Wanddicke im linken Ventrikel und der myocardiale Prote-ingehalt bestimmt.

Versuchsergebnis

| | PREVENTION (n : 17) | | | REGRESSION (n : 18) | | |
|---|---|---|---|---|---|---|
| | SHAM | CON | III | SHAM | CON | III |
| MAB | 92±2* | 153±4# | 118±3#* | 97±3* | 134±4 | 111±3#* |
| HG | 316±9* | 426±15# | 392±10#* | 303±10* | 398±11# | 353±15#* |
| δHG% | -- | +35 %# | +24 %#* | -- | +31 %# | +17 %#* |

p < 0,05 = * = gegen Kontrollgruppe; # = gegen Scheinkontrolle;

HG = Herzgewicht (mg/100 g Körpergewicht); MAB = mittlerer arterieller

Blutdruck (mmHg)

Die in dieser Tabelle angezeigten, signifikanten Veränderungen im δHG%-Wert von 24 % bzw. 17 % belegen die preventive sowie regressive Wirkung der Verbindung III in bezug auf eine cardiale Hypertrophie.

Die folgenden Beispiele geben die Anwendungsformen zur Behandlung der cardialen Hypertrophie nach der erfindungsgemäßen Methode an.

Beispiel 1

Herstellung des erfindungsgemäß verwendeten Mittels zur oralen Anwendung in der Behandlung der cardialen Hypertrophie.

1 000 Tabletten, die je 20 mg 2-n-Butyl-4-chloro-5-hydroxymethyl-1-[(2'-(1H-tetrazol-5-yl)-biphenyl-4-yl)-methyl]-imidazol-Kaliumsalz enthalten, werden mit den folgenden Hilfsmitteln hergestellt:

| 2-n-Butyl-4-chloro-5-hydroxymethyl-1-[(2'-(1H-tetrazol-5-yl)-biphenyl-4-yl)-methyl]-imidazol- | |
|---|---|
| Kaliumsalz | 20,0 g |
| Maisstärke | 140,0 g |
| Gelantine | 7,5 g |
| Mikrokristalline Cellulose | 2,5 g |
| Magnesiumstearat | 2,5 g |

2-n-Butyl-4-chloro-5-hydroxymethyl-1-[(2'-(1H-tetrazol-5-yl)-biphenyl-4-yl)-methyl]-imidazol-Kaliumsalz und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1 000 Tabletten gepreßt, wobei jede Tablette 20 mg des Angiotensin-II-Rezeptor-Antagonisten enthält. Diese Tabletten können zur Behandlung der cardialen Hypertrophie verwendet werden.

Beispiel 2

Analog Beispiel 1 werden 1 000 Tabletten hergestellt, die je 3 mg 2-n-Butyl-4-chloro-5-hydroxymethyl-1-[(2'-(1H-tetrazol-5-yl)-biphenyl-4-yl)-methyl]-imidazo1-Kaliumsalz enthalten, indem man von dieser Verbindung 3 g in den in Beispiel 1 beschriebenen Ansatz verwendet.

Beispiel 3

Gelatine-Kapseln, die je 20 mg 2-n-Butyl-4-chloro-5-hydroxymet hyl-1-[(2'-(1H-tetrazol-5-yl)-biphenyl-4-yl)-methyl]-imidazol-Kaliumsalz enthalten, werden mit der folgenden Mischung gefüllt:

| 2-n-Butyl-4-chloro-5-hydroxymethyl-1-[(2'-(1H-tetrazol-5-yl)-biphenyl-4-yl)-methyl]-imidazol- | |
|---|---|
| Kaliumsalz | 20 g |
| Kaliumstearat | 1 mg |
| Lactose | 214 mg |

Diese Kapseln können zur Behandlung der cardialen Hypertrophie verwendet werden.

Beispiel 4

Analog Beispiel 3 werden unter Verwendung von 3 g Wirkstoff Gelatine-Kapseln hergestellt, die je 3 mg 2-n-Butyl-4-chloro-5-hydroxymethyl-1-[(2'-(1H-tetrazol-5-yl)-biphenyl-4-yl)-methyl]-imidazol-Kaliumsalz enthalten.

Beispiel 5

Die Herstellung einer Injektionslösung zur Behandlung der cardialen Hypertrophie wird im folgenden beschrieben:

| 2-n-Butyl-4-chloro-5-hydroxymethyl-1-[(2'-(1H-tetrazol-5-yl)-biphenyl-4-yl)-methyl]-imidazol-Kaliumsalz | 1 g |
|---|---|
| Methylparaben | 5 g |
| Propylparaben | 1 g |
| Natriumchlorid | 25 g |
| Wasser für Injektion | 5 l |

2-n-Butyl-4-chloro-5-hydroxymethyl-1-[(2'-(1H-tetrazol-5-yl)-biphenyl-4-yl)-methyl]-imidazol-Kaliumsalz, die Konservierungsstoffe und Natriumchlorid werden in 3 l Wasser für Injektion gelöst und auf 5 l mit Wasser für Injektion aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

Beispiel 6

Tabletten, die zur Behandlung der cardialen Hypertrophie verwendet werden können, werden wie in Beispiel 1 beschrieben hergestellt, mit der Ausnahme, daß anstelle von 2-n-Butyl-4-chloro-5-hydroxymethyl-1-[(2'-(1H-tetrazol-5-yl)-biphenyl-4-yl)-methyl]-imidazol-Kaliumsalz 2-n-Butyl-5-carboxy-4-chloro-1-[(2'-(1H-tetrazol-5-yl)-biphenyl-4-yl)-methyl]-imidazol-Kaliumsalz oder
2-n-Butyl-1-[(2'-carboxy-biphenyl-4-yl)-methyl]-4-chloro-5-hydroxymethyl-imidazol-Kaliumsalz,
2-n-Butyl-5-carboxy-1-[(2'-carboxy-biphenyl-4-yl)-methyl]-4-chloro-imidazol-Kaliumsalz oder die entsprechenden Natriumsalze angewendet werden.

Beispiel 7

Eine Injektionslösung wird analog der in Beispiel 5 beschriebenen Vorschrift hergestellt mit der Ausnahme, daß anstelle von 2-n-Butyl-4-chloro-5-hydroxymethyl-1-[(2'-(1H-tetrazol-5-yl)-biphenyl-4-yl)-methyl]-imidazol-Kaliumsalz 2-n-Butyl-5-carboxy-4-chloro-1-[(2'-(1H-tetrazol-5-yl)-biphenyl-4-yl)-methyl]-imidazol-Kaliumsalz oder
2-n-Butyl-1-[(2'-carboxy-biphenyl-4-yl)-methyl]-4-chloro-5-hydroxymethyl-imidazol-Kaliumsalz,
2-n-Butyl-5-carboxy-1-[(2'-carboxy-biphenyl-4-yl)-methyl]-4-chloro-imidazol-Kaliumsalz oder die entsprechenden Natriumsalze angewendet werden.

**Patentansprüche**

1. Verwendung einer Verbindung aus der Gruppe
   2-n-Butyl-4-chloro-5-hydroxymethyl-1-[(2'-(1H-tetrazol-5-yl)-biphenyl-4-yl)-methyl]-imidazol,
   2-n-Butyl-5-carboxy-4-chloro-1-[(2'-(1H-tetrazol-5-yl)-biphenyl-4-yl)methyl]-imidazol,
   2-n-Butyl-1-[(2'-carboxy-biphenyl-4-yl)-methyl]-4-chloro-5-hydroxymethyl-imidazol und
   2-n-Butyl-5-carboxy-1-[(2'-carboxy-biphenyl-4-yl)-methyl]-4-chloro-imidazol oder dessen physiologisch verträglichen Salzes zur Herstellung eines Arzneimittels für die Behandlung der cardialen Hypertrophie.

2. Verwendung gemäß Anspruch 1, dadurch gekennzeichnet, daß
   2-n-Butyl-4-chloro-5-hydroxymethyl-1-[(2'-(1H-tetrazol-5-yl)-biphenyl-4-yl)-methyl]-imidazol oder
   2-n-Butyl-5-carboxy-4-chloro-1-[(2'-(1H-tetrazol-5-yl)-biphenyl-4-yl)methyl]-imidazol eingesetzt wird.

3. Verwendung gemäß Anspruch 1, dadurch gekennzeichnet, daß 2-n-Butyl-4-chloro-5-hydroxymethyl-1-[(2'-(1H-tetrazol-5-yl)-biphenyl-4-yl)-methyl]-imidazol eingesetzt wird.

**Claims**

1.  The use of a compound from the group comprising 2-n-butyl-4-chloro-5-hydroxymethyl-1-[(2'-(1H-tetrazol-5-yl)biphenyl-4-yl)methyl]imidazole
    2-n-butyl-5-carboxy-4-chloro-1-[(2'-(1H-tetrazol-5-yl)biphenyl-4-yl)methyl]imidazole
    2-n-butyl-1-[(2'-carboxybiphenyl-4-yl)methyl]-4-chloro-5-hydroxymethylimidazole and
    2-n-butyl-5-carboxy-1-[(2'-carboxybiphenyl-4-yl)-methyl]-4-chloroimidazole
    or its physiologically tolerable salt for the production of a pharmaceutical for the treatment of cardiac hypertrophy.

2.  The use as claimed in claim 1, wherein 2-n-butyl-4-chloro-5-hydroxymethyl-1-[(2'-(1H-tetrazol-5-yl)biphenyl-4-yl)methyl]imidazole or
    2-n-butyl-5-carboxy-4-chloro-1-[(2'-(1H-tetrazol-5-yl)biphenyl-4-yl)methyl]imidazole
    is employed.

3.  The use as claimed in claim 1, wherein 2-n-butyl-4-chloro-5-hydroxymethyl-1-[(2'-(1H-tetrazol-5-yl)-biphenyl-4-yl)methyl]imidazole is employed.

**Revendications**

1.  Utilisation d'un composé du groupe constitué par les 2-n-butyl-4-chloro-5-hydroxyméthyl-1-[(2'-(1H-tétrazol-5-yl)-biphényl-4-yl)méthyl]-imidazole,
    2-n-butyl-5-carboxy-4-chloro-1-[(2'-(1H-tétrazol-5-yl)-biphényl-4-yl)méthyl]-imidazole,
    2-n-butyl-1-[(2'-carboxy-biphényl-4-yl)méthyl]-4-chloro-5-hydroxyméthyl-imidazole,
    et
    2-n-butyl-5-carboxy-1-[(2'-carboxy-biphényl-4-yl)méthyl]-4-chloro-imidazole,
    ou d'un sel physiologiquement acceptable de ceux-ci pour préparer un médicament destiné au traitement de l'hypertrophie cardiaque.

2.  Utilisation selon la revendication 1, caractérisée en ce qu'on utilise
    le 2-n-butyl-4-chloro-5-hydroxyméthyl-1-[(2'-(1H-tétrazol-5-yl)-biphényl-4-yl)méthyl]-imidazole
    ou
    le 2-n-butyl-5-carboxy-4-chloro-1-[(2'-(1H-tétrazol-5-yl)-biphényl-4-yl)méthyl]-imidazole.

3.  Utilisation selon la revendication 1, caractérisée en ce qu'on utilise le 2-n-butyl-4-chloro-5-hydroxyméthyl-1-[(2'-(1H-tétrazol-5-yl)-biphényl-4-yl)méthyl]-imidazole.